(19) Europäisches Patentamt
European Patent Office
Office européen des brevets

(11) EP 0 451 055 B1

(12) **FASCICULE DE BREVET EUROPEEN**

(45) Date de publication et mention de la délivrance du brevet: **14.08.1996 Bulletin 1996/33**

(21) Numéro de dépôt: **91400910.5**

(22) Date de dépôt: **03.04.1991**

(51) Int. Cl.$^6$: **C07D 219/02**, G01R 33/24, C07D 221/16, C07D 471/16 // (C07D471/16, 221:00, 221:00, 221:00)

(54) **Utilisation en magnétométrie par résonance paramagnétique électronique (RPE) de dérivés de tétracyanoquinodiméthane**

Verwendung von Tetracyanchinodimethanderivaten in der paramagnetischen Elektronresonanz-Magnetometrie

Use of tetracyanoquinodimethane derivatives in electron paramagnetic resonance magnetometry

(84) Etats contractants désignés:
**CH DE GB IT LI NL**

(30) Priorité: **04.04.1990 FR 9004322**

(43) Date de publication de la demande:
**09.10.1991 Bulletin 1991/41**

(73) Titulaire: **COMMISSARIAT A L'ENERGIE ATOMIQUE**
**75015 Paris (FR)**

(72) Inventeur: **Moussavi, Mehdi**
**F-38120 Saint Egreve (FR)**

(74) Mandataire: **Des Termes, Monique et al**
**Société Brevatome**
**25, rue de Ponthieu**
**75008 Paris (FR)**

(56) Documents cités:
**FR-A- 2 603 384**

- **COMPTE RENDU DES SEANCES HEBDOMACLAIRS DE L'ACADEMIE DES SCIENCES, vol. 265, serie C, 2 octobre 1967, pages 688-690, Paris, FR; P. DUPUIS et al.: "Conductivité électrique de quelques complexes du tétracyanoquinodiméthane"**
- **MOLECULAR CRYSTALS AND LIQUID CRYSTALS, vol. 32, 1976, pages 209-213, Londres, GB; DELHAES et al.: "Etude comparative de la chaleur spécifique et du paramagnétisme de sels de TCNQ à cations dimagnétiques et radicalaires"**
- **TETRAHEDRON LETTERS, no. 38, 1977, pages 3325-3328, Oxford, GB; G. SAITO et al. : "Synthesis of anion-radical salts by hybride transfer reactions"**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 451 055 B1

Printed by Rank Xerox (UK) Business Services
2.13.0/3.4

## Description

La présente invention a pour objet l'utilisation de dérivés de tétracyanoquinodiméthane (TCNQ) en magnétométrie par résonance paramagnétique électronique (RPE).

De façon plus précise, elle concerne l'utilisation en magnétométrie RPE de composés organiques à transfert de charge du type $(A_a)^+$ $(TCNQ_b)$ • dans lesquels a=1, b=2 et A représente un cation dérivé d'une base aromatique hétérocyclique.

Les complexes à transfert de charge de ce type sont connus depuis le début des années 1960 et ont été très étudiés pour leurs propriétés conductrices de l'électricité. Des exemples de tels complexes sont décrits par exemple dans Nature, vol. 309, 1984, p. 119-126 ; Accounts of Chemical Research, vol. 12, n° 3, 1979, pages 79 à 86 ; dans FR-A-2 564 092 et dans les comptes rendus hebdomadaires des séances de l'Académie des Sciences, vol. 265, série C, 2 octobre 1967, pages 668-690, Paris, FR . P. Dupuis et al.

Généralement ces complexes organiques sont utilisés pour leurs propriétés conductrices ou semiconductrices. Toutefois, on a envisagé l'utilisation en magnétométrie RPE du tétracyanoquinodiméthane quinoléinium comme il est décrit dans FR-A- 2 603 384.

Delhaes et al dans Mol. Cryst. Liq. Cryst, 1976, vol 32, p. 209-213, ont étudié les propriétés physiques et structurales de sels de TCNQ à cations diamagnétiques tels que l'acridinium et le quinolinium, mais n'ont pas envisagé leur utilisation en magnétométrie par résonance paramagnétique électronique.

Pour une utilisation en magnétométrie RPE, les substances les plus appropriées sont celles qui présentent :

- une grande susceptibilité magnétique, (1 à $4.10^{-4}$ en uem/mol)
- un signal unique à bas champ (pas de couplage hyperfin), et
- une bonne stabilité de l'état solide de la substance dans la gamme de température d'utilisation allant de -40 à + 70°C.

Ces qualités se traduisent par le facteur de mérite du matériau qui peut être évalué à l'aide du procédé et du dispositif décrit dans le document FR-A- 2 634 556.

Par ailleurs, il est intéressant pour ces utilisations en RPE de trouver la substance paramagnétique dont la fabrication soit la plus aisée et la moins coûteuse, et qui nécessitera moins de puissance pour l'entretien de la résonance dans le magnétomètre.

Le facteur de mérite volumique du dérivé tétracyanoquinodiméthane-quinoléinium, déterminé en utilisant le procédé et le dispositif du document FR-A- 2 634 556 à une fréquence de 1,845MHz, est de $2,7.10^{-6}$. Cette valeur est obtenue pour la puissance haute fréquence maximum pouvant être fournie par le spectromètre, mais étant donné la conductivité électrique élevée de cette substance, la grande puissance HF nécessaire pour obtenir un bon signal rend critique le problème de couplage direct entre la bobine de détection et la bobine HF. Ainsi, le signal est distordu et le réglage du magnétomètre devient délicat.

De plus, la fabrication de cette substance paramagnétique nécessite des opérations complexes de purification et de recristallisation qui conduisent à des temps et des coûts de préparation élevés.

Aussi, des recherches ont été entreprises pour trouver d'autres substances paramagnétiques présentant un facteur de mérite au moins équivalent et des largeurs de raies égales ou plus faibles, mais nécessitant des puissance HF plus faibles pour entretenir la résonance paramagnétique.

La présente invention a précisément pour objet l'utilisation en RPE de dérivés de TCNQ présentant ces propriétés améliorées, qui répondent à la formule :

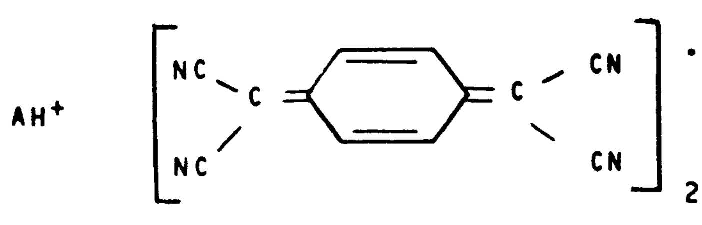

(I)

dans laquelle $AH^+$ est un cation choisi parmi les cations benzoquinoléinium, acridinium, phénantridinium, phénothiazinium, phénarsazinium et benzoisoquinoléinium,qui peuvent être éventuellement substitués par des radicaux alkyle de 1 à 8 atomes de carbone.

Ces substitutions éventuelles ne peuvent exister que sur les atomes de carbone des noyaux aromatiques, car pour une utilisation en magnétométrie RPE, il est important que l'atome d'azote du cation soit lié à un atome d'hydrogène. En effet, les dérivés N-alkylés de la quinoléine ou de l'acridine ont une conductivité trop forte pour cette application.

A titre d'exemple de dérivés de tétracyanoquinodiméthane conformes à l'invention, on peut citer :

(II)

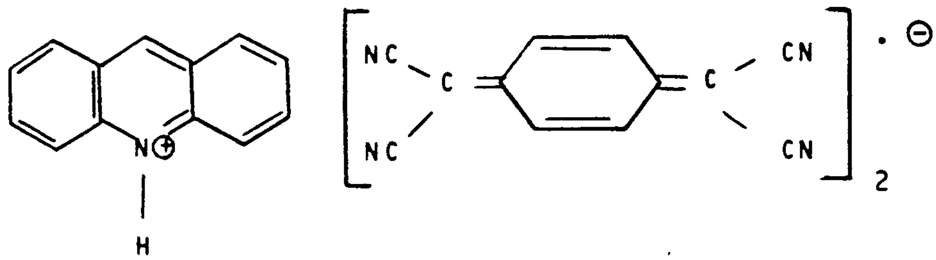

(III)

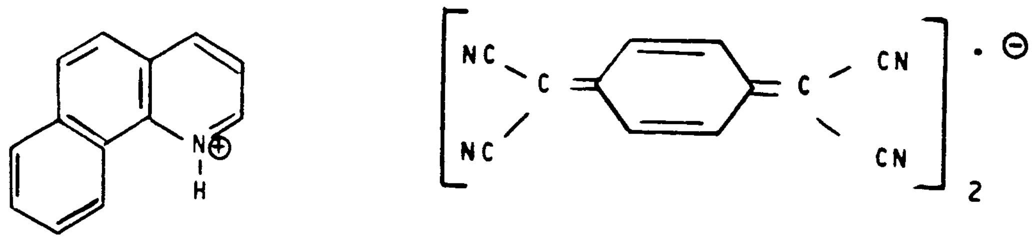

(IV)

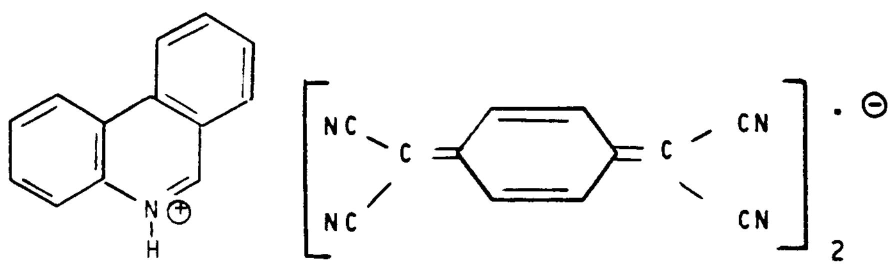

(V)

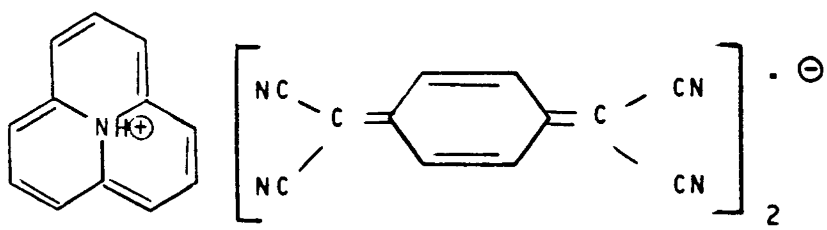

Dans le dérivé de l'invention, le fait de remplacer le cation quinoléinium par un cation ayant au moins trois noyaux aromatiques permet d'obtenir une plus grande compacité, d'avoir une plus grande extension du nuage d'électrons $\pi$ tout en gardant la structure plane de la molécule, et ceci se traduit par une meilleure dynamique de spin et des raies de résonance moins larges qui demandent de ce fait moins de puissance HF pour l'entretien de la résonance.

L'invention concerne également un procédé de préparation des dérivés de tétracyanoquinodiméthane répondant à la formule (I) donnée ci-dessus.

Ce procédé consiste à faire réagir un iodure de $AH^+$ avec du tétracyanoquinodiméthane.

Cette réaction peut être effectuée dans un solvant organique tel que l'acétonitrile, en ajoutant la solution d'iodure de $AH^+$ à une solution de TCNQ dans un solvant organique porté à l'ébullition.

Le produit de départ c'est-à-dire l'iodure de $AH^+$ peut être préparé en faisant réagir la base aromatique A correspondant au cation $AH^+$ avec de l'acide iodhydrique. Cette réaction peut être effectuée dans un solvant tel que l'éthanol.

L'invention concerne également un magnétomètre à résonance paramagnétique électronique utilisant comme substance présentant un moment magnétique électronique le dérivé de tétracyanoquinodiméthane de l'invention.

Avantageusement, ce magnétomètre comprend une enceinte contenant une substance présentant un moment magnétique électronique, un premier enroulement bobiné autour de cette enceinte et apte à produire une tension due

à une variation de flux magnétique résultant de la précession du moment magnétique électronique de cette substance autour d'un champ magnétique ambiant (H0), cette tension ayant une fréquence dite de Larmor égale à $\gamma H0/2\pi$ où $\gamma$ est le rapport gyromagnétique propre à la substance utilisée, un deuxième enroulement apte à créer un champ magnétique tournant (H1) à cette fréquence de Larmor pour entretenir la précession, et des moyens électroniques aptes, d'une part, à mesurer la fréquence du signal prélevé aux bornes du premier enroulement, ce qui donne le module du champ magnétique ambiant (H0) et, d'autre part, à délivrer le champ d'entretien (H1), et utilise comme substance présentant un moment magnétique électronique le dérivé de tétracyanoquinodiméthane de l'invention.

D'autres caractéristiques et avantages de l'invention apparaîtront mieux à la lecture des exemples suivants donnés bien entendu à titre illustratif et non limitatif, en référence au dessin annexé sur lequel :

- la figure 1 représente les courbes de dispersion de dérivés de TCNQ, et
- la figure 2 illustre de façon schématique un magnétomètre à RPE.

**<u>Exemple 1</u>** : <u>Préparation de l'acridinium bis tétracyanoquinodiméthane de formule</u> :

(II) 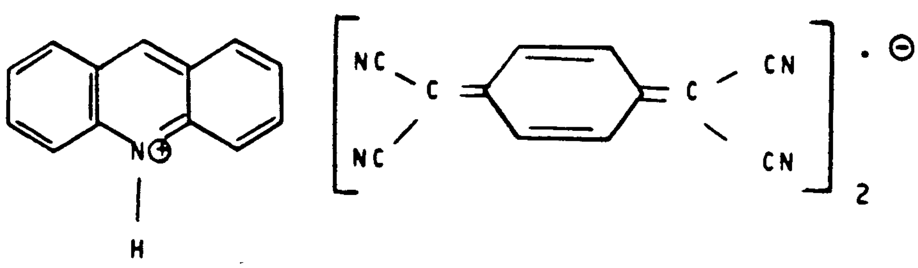

a) <u>Préparation de l'iodure d'acridinium.</u>

Cette réaction correspond au schéma réactionnel suivant :

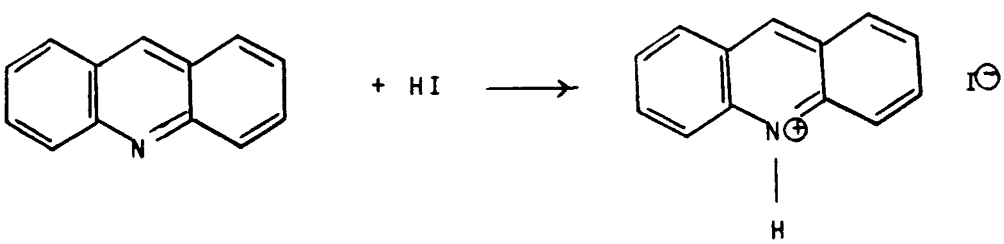


On ajoute goutte à goutte 1,5g (7,5mmol) d'acide iodhydrique à 65% à une solution de 1,35g (7,5mmol) d'acridine dans 20ml d'éthanol. Vers la fin de l'addition, un précipité orange apparaît. On ajoute alors de l'éthanol tout en chauffant à 50-60°C jusqu'à dissolution complète de ce précipité (environ 20ml d'éthanol). On laisse refroidir au réfrigérateur et on filtre l'iodure d'acridinium. On récupère ainsi 2,2g d'un solide microcristallin rouge orange, ce qui correspond à un rendement de 96%.

b) <u>Préparation de l'acridinium bis tétracyanoquinodiméthane.</u>

On fait réagir ensuite l'iodure d'acridinium obtenu dans l'étape a) avec du TCNQ, selon le schéma réactionnel suivant :

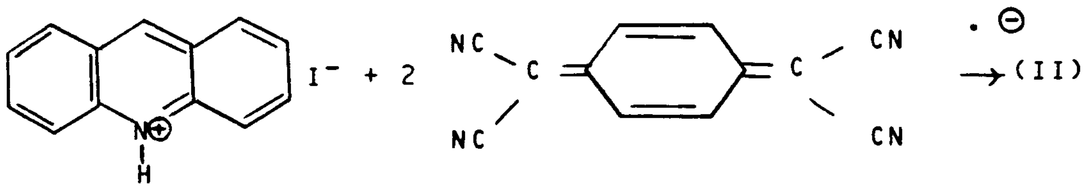

On ajoute une solution de 0,8g d'iodure d'acridinium dans 25ml d'acétonitrile à une solution de 1g (5mmol) de TCNQ pur dans 250ml d'acétonitrile bouillant, en opérant au reflux et sous atmosphère d'argon. Après 30min de reflux, on laisse refroidir le mélange réactionnel sous argon et après 2h de repos, on filtre le précipité et on récupère ainsi 1g d'acridinium bis tétracyanoquinodiméthane que l'on lave à l'éther ; le rendement est de 34%.

**<u>Exemple 2</u>** : <u>Préparation du benzoquinoléinium bis tétracyanoquinodiméthane de formule</u> :

(III) 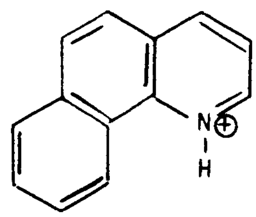

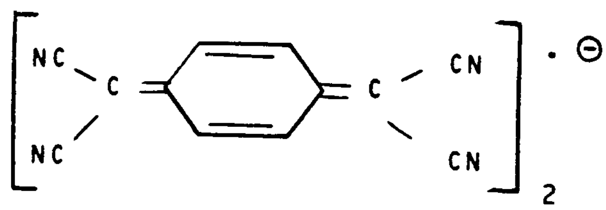

On suit le même mode opératoire que dans l'exemple 1 pour préparer ce dérivé de TCNQ, sauf que l'on utilise dans l'étape a) pour la préparation de l'iodure, (7,5mmol) de benzoquinoléine au lieu de 7,5mmol d'acridine.

On obtient ainsi le benzoquinoléinium bis tétracyanoquinodiméthane avec un rendement de 30%.

**<u>Exemple 3</u>**.

Dans cet exemple, on détermine les propriétés des dérivés de TCNQ préparés dans les exemples 1 et 2, pour la magnétométrie RPE.

Dans ce but, on détermine le facteur de mérite, la largeur de raie, la puissance HF nécessaire pour entretenir la résonance et la modulation BF, en utilisant le spectromètre décrit dans FR-A- 2 634 556.

Les résultats obtenus sont donnés dans le tableau qui suit.

TABLEAU

| Dérivé de TCNQ | Facteur de mérite | Largeur de raie (A/m) | Puissance HF (A/m) | Modulation BF (A/m) |
|---|---|---|---|---|
| Quinoléinium-bis-TCNQ | $2,7.10^{-6}$ | 20,2 | 22,5 | 9,5 |
| Acridinium-bis-TCNQ (ex 1) | $2,9.10^{-6}$ | 18,7 | 11,2 | 10 |
| Benzoquinoléinium-bis-TCNQ (ex 1) | $2,0.10^{-6}$ | 18 | 9 | 10 |

Dans ce tableau, on a donné à titre comparatif les résultats obtenus avec le quinoléinium-bis-tétracyanoquinodiméthane obtenu à partir d'iodure de quinoléinium en suivant le même procédé que dans l'invention.

Au vu de ces résultats, on constate que le facteur de mérite croît lorsque l'on passe de la quinoléine à l'acridine, mais qu'il est plus faible avec la benzoquinoléine. En revanche, la largeur de raie diminue lorsque l'on passe de la quinoléine à l'acridine et à la benzoquinoléine. Il en est de même de la puissance HF qui diminue de façon importante alors que la modulation BF est équivalente pour les trois dérivés.

Ainsi, les dérivés de l'invention sont plus intéressants que le quinoléinium bis TCNQ. De plus, leur préparation est moins coûteuse car les iodures d'acridinium et de benzoquinoléinium se préparent plus facilement, en quelques minutes, même en grandes quantités et ne nécessitent aucune purification particulière, ce qui n'est pas le cas de l'iodure de quinoléinium.

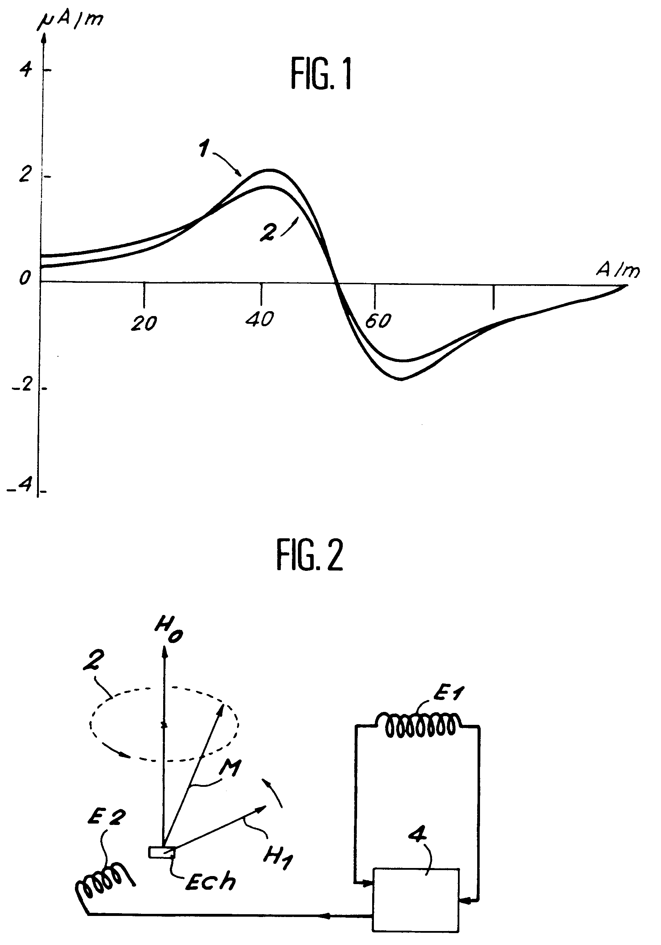

Sur la figure 1, on a représenté schématiquement la courbe de dispersion du dérivé acridinium-bis-TCNQ de l'invention (courbe 1) et la courbe de dispersion du dérivé quinoléinium-bis-TCNQ (courbe 2) tracées avec le spectromètre décrit dans FR-A- 2 634 556 à une fréquence de 1,846MHz. Sur cette figure, on voit que le dérivé de l'invention donne de meilleurs résultats.

Sur la figure 2, on a représenté de façon schématique un magnètomètre à RPE utilisant les dérivés de l'invention destiné plus particulièrement à la mesure de champs magnétiques faibles, qui peut trouver des applications en géophysique, en prospection minière, en détection militaire et spatiale, et aussi en robotique industrielle.

L'existence du spin de l'électron et, par conséquent, d'un moment magnétique non nul, est la cause du mouvement de précession de l'électron autour de l'axe d'un champ magnétique ambiant. La fréquence de précession, dite de Larmor, est déterminée par la relation suivante :

$$F = \gamma H0/2\pi$$

où H0 est le module du champ ambiant et le rapport gyromagnétique de l'électron.

Le principe d'un magnétomètre à résonance paramagnétique électronique consiste à détecter la variation de flux magnétique engendrée par la précession du moment magnétique d'une substance paramagnétique. Pour cela on utilise un enroulement sensible à l'induction variable, laquelle fait apparaître, aux bornes de cet enroulement, une tension alternative qui est à la fréquence de Larmor. La mesure de cette fréquence donne la valeur du champ.

La figure 2 illustre ce principe. Le mouvement de précession de l'aimantation macroscopique M d'un échantillon de substance paramagnétique Ech autour de l'axe du champ ambiant H0 est symbolisée par le cercle 2. Cette aimantation est la somme des contributions alimentaires des moments magnétiques des électrons de la substance. Aux bornes d'un enroulement E1 apparaît une tension électrique dont l'amplitude est proportionnelle au carré du module du champ H0 et dont la fréquence est égale à la fréquence de Larmor. Un circuit de mesure permet de mesurer ces deux grandeurs.

Pour entretenir le mouvement de précession de l'aimantation macroscopique, il faut entraîner celle-ci par un champ tournant H1 à la fréquence de Larmor. Ce champ doit avoir une composante perpendiculaire à H0. Pour cela, on utilise un second enroulement E2 qui est alimenté par le circuit 4, par une tension à une fréquence aussi voisine que possible de la fréquence de Larmor.

Ce magnétomètre est donc un appareil qui mesure la fréquence de la tension induite par la précession de l'aimantation et qui, de plus, engendre un champ d'entraînement dont la fréquence doit être en permanence liée au module du champ ambiant.

Conformément à l'invention, l'échantillon de substance paramagnétique utilisé dans ce magnétomètre est constitué par un dérivé de tétracyanoquinodiméthane de formule (I).

## Revendications

1. Utilisation en magnétométrie par résonance paramagnétique électronique (RPE) d'un dérivé de tétracyanoquinodiméthane (TCNQ) répondant à la formule :

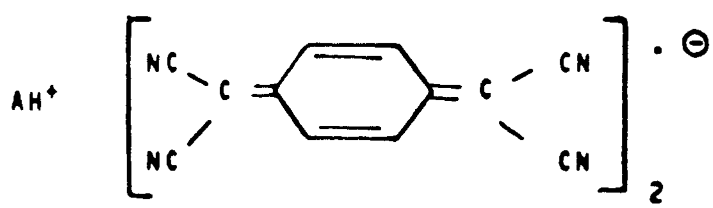

(I)

dans laquelle $AH^+$ est un cation choisi parmi les cations benzoquinoléinium, acridinium, phénantridinium, phénothiazinium, phénarsazinium et benzoisoquinoléinium, qui peuvent être éventuellement substitués par des groupes alkyle de 1 à 8 atome de carbone, sur les atomes de carbon de leurs noyaux aromatiques, ou le cation de formule :

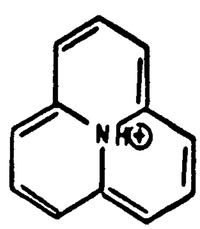

2. Utilisation selon la revendication 1, caractérisé en ce que $AH^+$ représente :

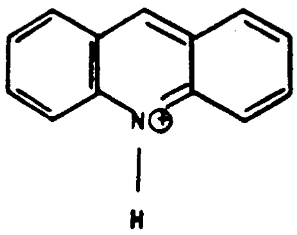

3. Utilisation selon la revendication 1, caractérisé en ce que $AH^+$ représente :

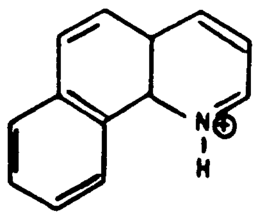

4. Utilisation selon la revendication 1, caractérisé en ce que $AH^+$ représente :

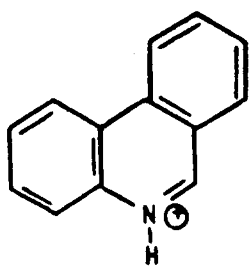

5. Magnétomètre à résonance paramagnétique électronique, comprenant une enceinte contenant une substance présentant un moment magnétique électronique, un premier enroulement (E1) bobiné autour de cette enceinte et apte à produire une tension due à une variation de flux magnétique résultant de la précession du moment magnétique électronique de cette substance autour d'un champ magnétique ambiant (H0), cette tension ayant une fréquence dite de Larmor égale à $\gamma H0/2\pi$ où $\gamma$ est le rapport gyromagnétique propre à la substance utilisée, un deuxième enroulement (E2) apte à créer un champ magnétique tournant (H1) à cette fréquence de Larmor pour entretenir la précession, et des moyens électroniques aptes, d'une part, à mesurer la fréquence du signal prélevé aux bornes du premier enroulement, ce qui donne le module du champ magnétique ambiant (H0) et, d'autre part, à délivrer le champ d'entretien (H1), caractérisé en ce que la substance présentant un moment magnétique électronique est un dérivé de tétracyanoquinodiméthane selon l'une quelconque des revendications 1 à 4.

## Claims

1. Use in magnetometry by electronic paramagnetic resonance (EPR) of a tetracyanoquinodimethane (TCNQ) derivative of formula:

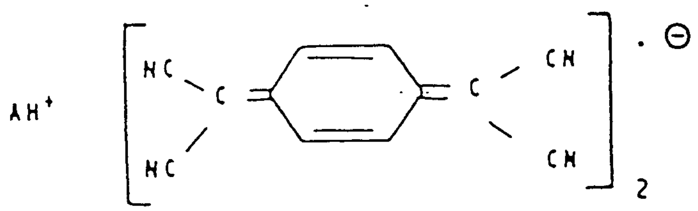

(I)

in which $AH^+$ is a cation chosen from among benzoquinolinium, acridinium, phenantridinium, phenothiazinium, phenarsazinium and benzoisoquinolinium cations, optionally substituted by alkyl groups with 1 to 8 carbon atoms on the carbon atoms of their aromatic nuclei, or the cation of formula:

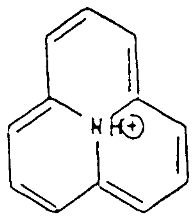

2. Use according to claim 1, characterized in that $AH^+$ represents:

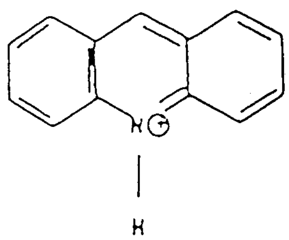

3. Use according to claim 1, characterized in that $AH^+$ represents:

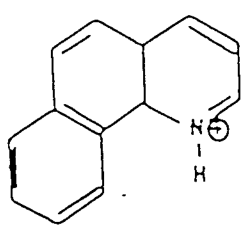

**4.** Use according to claim 1, characterized in that $AH^+$ represents:

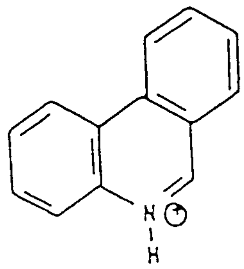

**5.** Electronic paramagnetic resonance magnetometer having an enclosure containing a substance with an electronic magnetic moment, a first winding (E1) wound around said enclosure and able to produce a voltage due to the variation of the magnetic flux resulting from the precession of the electronic magnetic moment of said substance around an ambient magnetic field (H0), said voltage having a Larmor frequency equal to $\gamma H0/2\pi$ in which $\gamma$ is the gyromagnetic ratio of the substance used, a second winding (E2) able to produce a rotary magnetic field (H1) at the Larmor frequency for maintaining the precession, and electronic means able, on the one hand, to measure the frequency of the signal taken at the terminals of the first winding, which gives the modulus of the ambient magnetic field (H0) and, on the other hand, to supply the maintenance field (H1), characterized in that the substance having an electronic magnetic moment is a tetracyanoquinodimethane derivative according to any one of the claims 1 to 4.

**Patentansprüche**

**1.** Verwendung eines Derivats des Tetracyanochinodimethans (TCNQ) entsprechend der Formel:

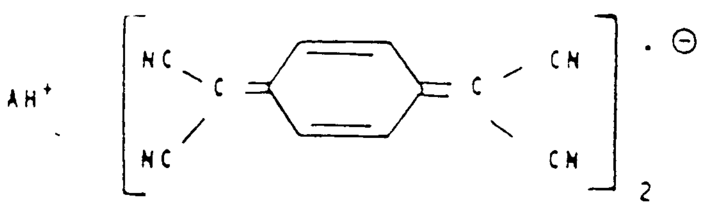 (I)

in der Magnetometrie durch paramagnetische Elektronenresonanz (EPR),

worin $AH^+$ ein unter den Benzochinolinium-, Acridinium-, Phenanthridinium-, Phenothiazinium-, Phenarsazinium- und Benzoisochinolinium-Kationen, die gegebenenfalls an den Kohlenstoffatomen ihrer aromatischen Kerne durch Alkylgruppen mit 1 bis 8 Kohlenstoffatomen substituiert sein können, gewähltes Kation ist oder das Kation der Formel:

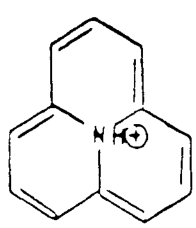

2. Verwendung nach Anspruch 1, dadurch gekennzeichnet, daß $AH^+$ steht für:

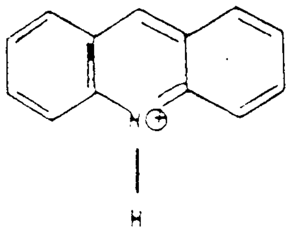

3. Verwendung nach Anspruch 1, dadurch gekennzeichnet, daß $AH^+$ steht für:

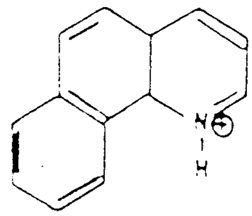

4. Verwendung nach Anspruch 1, dadurch gekennzeichnet, daß $AH^+$ steht für:

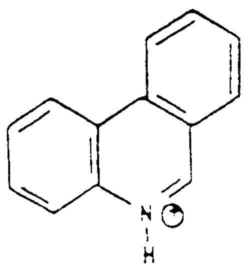

5. Magnetometer mit paramagnetischer Elektronenresonanz, umfassend einen Behälter, der eine ein elektronisches magnetisches Moment besitzende Substanz enthält, eine erste Wicklung (E1), um diesen Behälter gewickelt und geeignet, eine Spannung zu erzeugen, die auf einer aus der Präzession des elektronischen magnetischen Moments dieser Substanz um ein magnetisches Umgebungsfeld (H0) resultierenden Veränderung des magnetischen Flusses beruht, wobei diese Spannung eine als Larmor-Frequenz bezeichnete Frequenz besitzt, die gleich $\gamma H0/2\pi$ ist, wo $\gamma$ das für die verwendete Substanz eigentümliche gyromagnetische Verhältnis ist, eine zweite Wicklung (E2), geeignet, ein mit dieser Larmor-Frequenz rotierendes magnetisches Feld (H1) zu erzeugen, um die Präzession aufrechtzuerhalten, und elektronische Einrichtungen, geeignet, einerseits die Frequenz des an den Polen der ersten Wicklung abgenommenen Signals zu messen, was den Betrag des magnetischen Umgebungsfeldes (H0) gibt, andererseits das Aufrechterhaltungs-Feld (H1) zu liefern, dadurch gekennzeichnet, daß die ein elektronisches magnetisches Moment besitzende Substanz ein Derivat des Tetracyanochinodimethans nach einem der Ansprüche 1 bis 4 ist.

FIG. 1

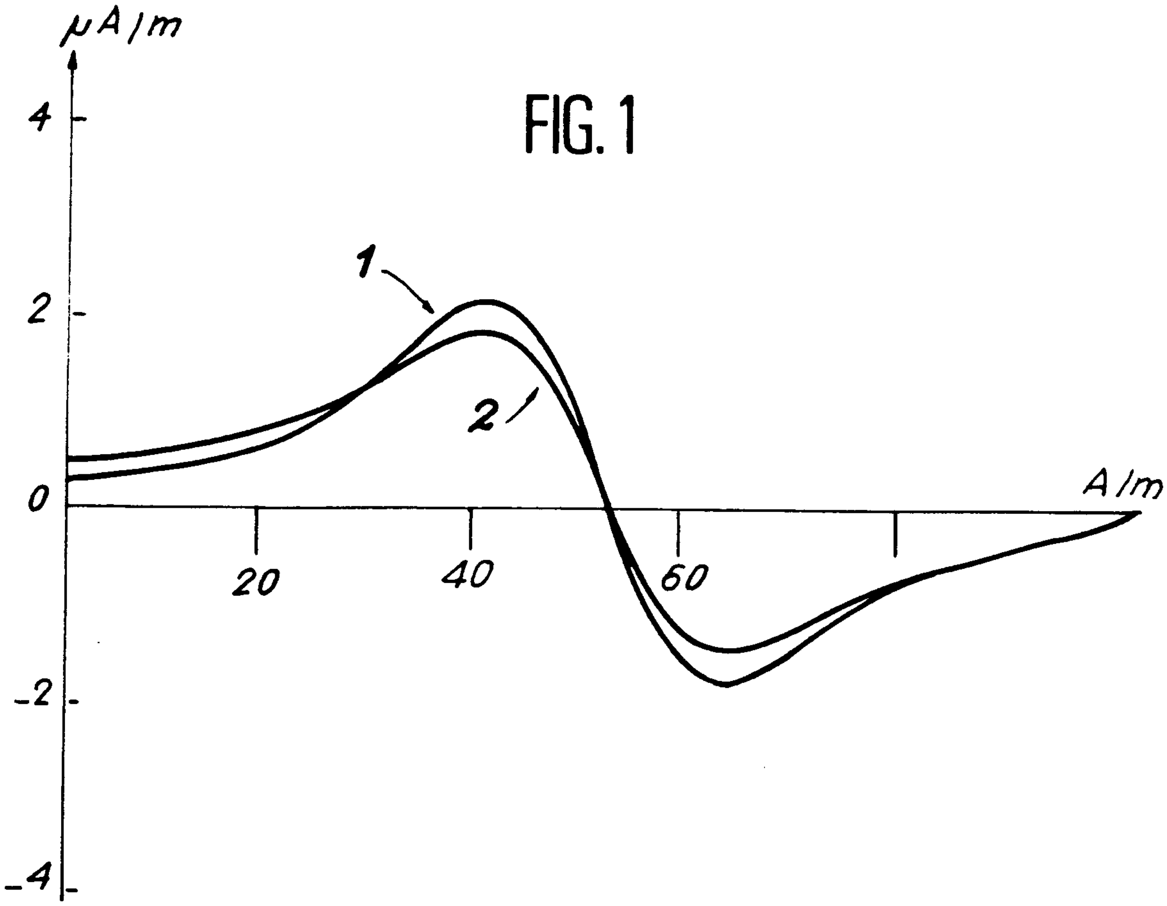


FIG. 2